(19) Europäisches Patentamt
European Patent Office
Office européen des brevets

(11) Veröffentlichungsnummer: **0 215 457 B1**

# (12) EUROPÄISCHE PATENTSCHRIFT

(45) Veröffentlichungstag der Patentschrift: **12.06.91**

(51) Int. Cl.5: **G01N 33/543**, //G01N33/74

(21) Anmeldenummer: **86112667.0**

(22) Anmeldetag: **12.09.86**

Die Akte enthält technische Angaben, die nach dem Eingang der Anmeldung eingereicht wurden und die nicht in dieser Patentschrift enthalten sind.

## (54) Verfahren zur Bestimmung eines Partners einer Immunreaktion.

(30) Priorität: **12.09.85 DE 3532626**

(43) Veröffentlichungstag der Anmeldung:
**25.03.87 Patentblatt 87/13**

(45) Bekanntmachung des Hinweises auf die Patenterteilung:
**12.06.91 Patentblatt 91/24**

(84) Benannte Vertragsstaaten:
**AT BE CH DE FR GB IT LI LU NL SE**

(56) Entgegenhaltungen:
**EP-A- 0 132 948** **EP-A- 0 133 272**
**EP-A- 0 171 946** **DE-A- 2 062 558**
**DE-A- 2 529 937** **DE-A- 3 500 190**
**FR-A- 2 299 645** **US-A- 4 357 310**

**R.J. CERESA "Block and graft copolymerization", Teil 2, 1976, Seiten 1-103, John Wiley and Sons, London, GB;**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Schmitt, Urban**
**Waldstrasse 36**
**W-8125 Oberhausen(DE)**
Erfinder: **Kleinhammer, Gerd, Dr. rer. nat.**
**Kreuzeckstrasse 3**
**W-8132 Tutzing(DE)**
Erfinder: **Deeg, Rolf, Dr. rer. nat.**
**Hirtenstrasse 7**
**W-8139 Bernried(DE)**

(74) Vertreter: **Weickmann, Heinrich, Dipl.-Ing. et al**
**Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820**
**W-8000 München 86(DE)**

EP 0 215 457 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Bestimmung eines Partners einer Immunreaktion nach dem Immunoassay-Prinzip bei einer Temperatur zwischen 15 und 40 °C, wobei einer der Reaktionspartner in fester Phase vorliegt.

Verfahren zur Immunbestimmung sind weit verbreitet. Dabei gibt es sowohl Verfahren in homogener als auch in heterogener Phase. Bei der Ausführungsform mit der heterogenen Phase wird einer der Reaktionspartner an einen Träger gebunden. Zur Durchführung der Immunbestimmung in heterogener Phase sind nun verschiedene Verfahrensvarianten bekannt, z. B. das Sandwich-Verfahren, das indirekte Verfahren und das Kompetitionsverfahren. Beim Sandwich-Verfahren wird ein Antikörper an den Träger gebunden, die Testlösung wird zugegeben, wobei das in der Testlösung enthaltene spezifische Antigen an den Antikörper gebunden wird. Dann wird ein markierter spezifischer Antikörper für den Antigen-Antikörper-Komplex, oder für Teile des Komplexes, zugegeben, der dann an den Komplex bindet. Über den markierten Antikörper kann man dann die Menge an Antigen berechnen. Beim indirekten Verfahren wird ein Antigen an dem Trägermaterial gebunden. Man gibt Testlösung dazu, wobei der in der Testlösung enthaltene, für das adsorbierte Antigen spezifische Antikörper mit dem Antigen reagiert. Bei Zugabe eines markierten Antiglobulins bindet das Antiglobulin an den Antigen-Antikörper-Komplex und die Menge des unbekannten Antikörpers im Testserum kann wiederum über das markierte Antiglobulin bestimmt werden. Bei dem Kompetitionsverfahren wird einer der gebundenen Partner der Immunreaktion an das Trägermaterialgebunden. Man gibt dann eine Lösung dazu, die sowohl den in unbekannter Menge vorliegenden anderen Partner der Immunreaktion enthält, als auch eine bekannte Menge an markiertem anderem Partner der Immunreaktion. Beide Partner, der markierte und der unmarkierte, konkurrieren um die Bindungsstellen des am Träger gebundenen Partners der Immunreaktion. Zu einer zweiten Probe gibt man eine Standardlösung dazu, die nur markierten Partner enthält. Man kann nun durch Bestimmung der markierten Partner in Standard und Probe aus der Differenz die Menge an unbekanntem Reaktionspartner berechnen.

Weitere Möglichkeiten zur Bestimmung ergeben sich bei einem mindestens bivalenten Antigen mit Hilfe von drei Rezeptoren, die Antikörper oder Antikörperfragmente sein können. Bei der Durchführung eines Immunoassays mit drei Rezeptoren in heterogener Phase ist einer der drei Rezeptoren immer unlöslich, während die anderen zwei löslich sind, wobei einer der beiden löslichen Rezeptoren markiert ist, während der andere nicht markiert ist. Der unlösliche Rezeptor ist dann gegen den nicht markierten löslichen Rezeptor gerichtet. Zur Durchführung der Bestimmung sind verschiedene Verfahrensvarianten möglich, sowohl einstufige, als auch mehrstufige, in denen die Zugabe der einzelnen Rezeptoren variiert werden kann.

Eine weitere Verfahrensvariante besteht darin, das zu bestimmende Antigen in der Probe im ersten Schritt gleichzeitig mit einem löslichen, nicht markierten Rezeptor und einem löslichen markierten Rezeptor in Lösung zusetzen und dann den entstehenden löslichen Sandwich-Komplex in einem zweiten Schritt durch Binden an einen unlöslichen Rezeptor unlöslich zu machen.

Zur Markierung der Reaktionspartner sind verschiedene Möglichkeiten bekannt. So kann einer der Reaktionspartner radioaktiv markiert werden, wobei dann die gemessene Radioaktivität eine Berechnung des zu bestimmenden Reaktionspartners erlaubt. Weiterhin kann mit einem Enzym markiert werden. Es wird dann nach Durchführung der üblichen Schritte der Immunbestimmung ein Substrat für das Enzym, das als Markierung verwendet wird, zugegeben und dann über dessen Reaktion die Konzentration des zu bestimmenden Reaktionspartners berechnet. Eine weitere Möglichkeit ist die Markierung mit einer fluoreszierenden Substanz, wobei die Fluoreszenz dann direkt bestimmt werden kann oder mit einem Farbstoff, wobei die Auswertung spektro- oder photometrisch erfolgt.

Immunassays weisen wachsende Bedeutung auf, da sie eine sehr große Empfindlichkeit haben und mit Hilfe dieser Methode Substanzen bis in den Picogrammbereich erfaßt werden können. Immunbestimmungen in heterogener Phase werden jedoch in unterschiedlichem Ausmaß durch nicht Analyt-spezifische Interferenzen, sogenannte unspezifische Störungen, verfälscht, die schon als "Matrixeffekt", "Background" und "unspezifische Bindung" bezeichnet wurden. Besonders störend macht sich bemerkbar, daß die Wiederfindung der zu bestimmenden Substanz bei Plasmaproben deutlich geringer als bei Serumproben ist. Diese unspezifischen Störungen sind teilweise auf unbekannte Probenbestandteile zurückzuführen. Ebenso kann sich die Empfindlichkeit der Immunbestimmung durch unspezifische Wechselwirkungen der Störfaktoren mit den eingesetzten Immunreagenzien oder mit dem Analyten verschlechtern. Es konnte festgestellt werden, daß unspezifische Störungen bei Immunbestimmungen im Plasma stärker auftreten als bei Immunbestimmungen im Serum. Deshalb ist auch die Wiederfindung bei Bestimmungen im Plasma wesentlich geringer als im Serum, während bei kompetitiven Tests die Wiederfindung im Plasma zu hoch ist. Die Ursache dieser Störung wurde daher auf die Gerinnungsproteine, die im Plasma noch vorhanden

sind, im Serum jedoch nicht, zurückgeführt. Die Gerinnungsproteine lagern sich an die Oberfläche des Trägermaterials an und behindern damit die Antigen-Antikörper-Reaktion bzw. die Antigen-Konjugatreaktion.

Es war bisher bekannt, zur Verringerung unspezifischer Wechselwirkungen bei Immunbestimmungen dem Inkubationsmilieu bestimmte Detergenzien zuzusetzen. Ein Nachteil dieser Methode besteht jedoch darin, daß auch die erwünschte, d. h. die analytspezifische Antigen-Antikörper-Reaktion in ihrem Ausmaß beeinflußt wird, woraus wiederum eine Abnahme der Empfindlichkeit resultiert. Außerdem trat bei der bekannten Anwendung von Detergenzien das Problem auf, daß bei Solid-phase- Immunoassays die oberflächenaktive Substanz das adsorptiv gebundene Molekül von der festen Oberfläche verdrängen konnte. Die Funktion des Immunoassays wird dadurch entscheidend beeinträchtigt.

So war bekannt, daß Detergenzien wie Tween® 20 im Inkubationspuffer die Plasmagängigkeit herstellen. Detergenzien dieser Art haben jedoch den Nachteil, daß sie in Konzentrationen, die höher sind als 0,01 %, den an das Trägermaterial gebundenen Reaktionspartner ablösen, was wiederum zu einer negativen Beeinflussung der Immunbestimmung führt.

Weiterhin war aus DE-A- 35 00 190 bekannt, daß immunochemische Reaktionen in Gegenwart eines relativ hydrophilen Detergens mit praktisch neutralem pH und einem HLB-Wert von wenigstens etwa 20 beschleunigt werden können. Als Detergentien sind hier anionische Substanzen wie Alkylbenzolsulfonate genannt. Auch der Zusatz dieser Detergentien befriedigt noch nicht.

Überdies, war aus FR-A-2 299 645 bekannt, daß die Verwendung von nichtionischen Block-Copolymeren in einem homogenen Immunverfahren den Grad an Agglutination des Komplexes, der sich aus Antigen und Antikörper bildet, steigern kann.

Es war nun ein Ziel der Erfindung, ein Verfahren zu finden, bei dem nicht analytspezifische und daher unerwünschte unspezifische Störungen bei Immunbestimmungen in heterogener Phase unterdrückt werden. Ferner war es ein Ziel, ein derartiges Verfahren zu finden, bei dem weder die Reaktion zwischen Antikörper und Antigen/Hapten beeinträchtigt wird, noch adsorptiv gebundene Reaktionsteilnehmer von dem Träger desorbiert werden, noch, wenn als Markierung enzymmarkierte Immunreagenzien verwendet werden, die enzymatische Aktivität reduziert wird. Ein weiteres Ziel der Erfindung ist auch eine Beschleunigung der Immunreaktion und damit eine Verkürzung der Analysendauer.

Zur Erreichung dieser Ziele schlägt die Erfindung ein Verfahren zur Bestimmung eines Partners einer Immunreaktion nach dem Immunoassay-Prinzip bei einer Temperatur zwischen 15 und 40 °C vor, wobei einer der Reaktionspartner in fester Phase vorliegt, das dadurch gekennzeichnet ist, daß man als oberflächenaktives Mittel nichtionische Block-Copolymere auf Basis von Alkylenoxiden mit 2 bis 4 C-Atomen, die gegebenenfalls ein Alkylendiamin mit 2 bis 4 C-Atomen als Zentralmolekül enthalten, mit einem HLB-Wert von mehr als 20 in einer Menge von 0,1 bis 2 Gew.-%, bezogen auf das Gewicht des Reaktionsansatzes zusetzt.

Überraschenderweise wurde festgestellt, daß Detergenzien mit einem HLB-Wert, der größer ist als 20, unerwünschte Reaktionen bei Immunbestimmungen unterdrücken können und gleichzeitig weder die Reaktion zwischen Antikörper und Antigen bzw. Hapten beeinträchtigen, noch eine Desorption der adsorptiv gebundenen Reaktionspartner bewirken. Auch eine Beeinflussung der Enzymaktivität wurde nicht festgestellt. Darüberhinaus führt der Zusatz des erfindungsgemäßen Mittels zu einer Beschleunigung der Immunreaktion.

Bevorzugte Blockcopolymere sind Verbindungen der allgemeinen Formel I

$$HO(CH_2CH_2O)_a(\underset{\displaystyle CH_3}{\underset{|}{C}}HCH_2O)_b(CH_2CH_2O)_aH,$$

bei denen a einen Wert zwischen 30 und 150 und besonders bevorzugt zwischen 60 und 100 und b einen Wert zwischen 15 und 60 und besonders bevorzugt zwischen 25 und 40 annehmen kann. Diese Verbindungen werden auch als Poloxamere bezeichnet. Eine Handelsbezeichnung ist z. B. Pluronic$^R$.

Bei der Gruppe der Poloxamere haben sich als besonders geeignet Verbindungen erwiesen, die einen Anteil an hydrophilem Polyoxyethylen im Gesamtmolekül von 60 bis 90 % haben.

Weiterhin bevorzugt sind Blockcopolymere mit der allgemeinen Formel II

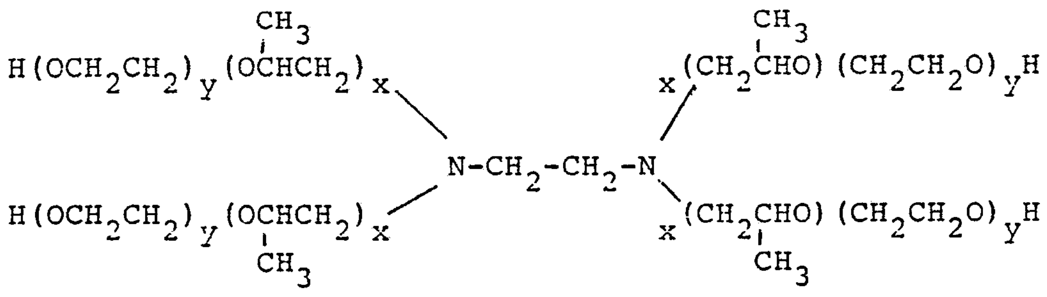

die als Poloxamine bezeichnet werden, worin x die Werte 10 bis 50, besonders bevorzugt 15 bis 30 und y die Werte 30 bis 150, besonders bevorzugt 40 bis 120 annehmen kann, verwendet. Eine Handelsbezeichnung für solche Poloxamine ist z. B. Tetronic$^R$. Unter den obengenannten oberflächenaktiven Mitteln werden solche am meisten bevorzugt, deren HLB-Wert zwischen 24 und 30 liegt.

Besonders bevorzugt werden bei dem Verfahren der vorliegenden Erfindung Poloxamine mit einem Anteil an hydrophilen Polyoxyethylengruppen von 50 bis 90% verwendet.

Der Zusatz des oberflächenaktiven Mittels gemäß der Erfindung bringt sowohl Vorteile beim Zusatz zu Plasma als auch beim Zusatz zu Serum. In beiden Fällen tritt eine Beschleunigung der Reaktion auf sowie eine Verhinderung der unspezifischen Störungen, insbesondere bei den hierfür besonders anfälligen Bestimmungen im Plasma.

Das erfindungsgemäße Verfahren ist auf alle Plasmaarten anwendbar. So kann es sowohl für Plasma, das zur Stabilisierung mit EDTA versetzt wurde, als auch für Plasma, das mit Heparin oder Citrat versetzt wurde, verwendet werden.

Der Zusatz des oberflächenaktiven Mittels kann bei jeder für die Bestimmung verwendeten Lösung erfolgen. Bei einem zweistufigen Verfahren werden besonders gute Ergebnisse erhalten, wenn beiden Puffern, also für jede der beiden Stufen, das Detergenz zugesetzt wird. Dazu wird die feste Phase, die den einen Reaktionspartner trägt, zuerst mit einer ersten Inkubationslösung behandelt, die Puffer und Zusatzstoffe, wie z. B. IgG enthält. Danach wird gewaschen und anschließend in einer zweiten Stufe die Konjugatlösung zugesetzt und erneut inkubiert.

Die Konjugatlösung enthält Konjugat und Puffer sowie gegebenenfalls weitere übliche Zusätze. Das Konjugat selbst besteht aus einem immunologischen Reaktionspartner für die zu bestimmende Substanz oder für den Festphasenpartner, z. B. einem Antikörper gegen das zu bestimmende Antigen in markiertem Zustand. Die Probelösung wird dabei zweckmäßig in den Inkubationspuffer gegeben. Gute Ergebnisse werden aber auch dann erzielt, wenn der Zusatz der oberflächenaktiven Substanz zur Inkubationslösung oder zur Konjugatlösung erfolgt.

Der Zusatz des oberflächenaktiven Mittels zur Probelösung selbst ist weniger günstig. In der fertigen Inkubationslösung muß ja eine ausreichend hohe Konzentration desselben vorliegen. Wenn nun das Mittel über die Probelösung zugegeben wird, die in nur sehr geringer Menge verwendet wird, muß die Konzentration an oberflächenaktivem Mittel darin sehr hoch sein. Bei sehr hohen Konzentrationen könnten jedoch unerwünschte Wirkungen auftreten.

Zur Durchführung des erfindungsgemäßen Verfahrens wird ein Reagenz sowie ein oberflächenaktives Mittel verwendet, das dadurch gekennzeichnet ist, daß es als oberflächenaktives Mittel nichtionische Block-Copolymere auf Basis von Alkylenoxiden mit 2 bis 4 C-Atomen, die gegebenenfalls ein Alkylendiamin mit 2 bis 4 C-Atomen als Zentralmolekül enthalten, mit einem HLB-Wert von mehr als 20 in einer Menge von 0,1 bis 2 Gew.-% enthält. Dieses Reagenz kann außerdem noch an sich übliche Inhaltsstoffe enthalten. Zweckmäßig enthält es noch Puffersubstanzen, beispielsweise Phosphatpuffer, Citratpuffer, Boratpuffer und dergleichen oder/und Rinderserumalbumin oder/und Konversierungsmittel. Wird als Markierung bei der Immunreaktion ein Enzym verwendet,so enthält das Reagenz noch ein System zum Nachweis der Enzymaktivität.

Bevorzugt enthält das Reagenz als oberflächenaktives Mittel eine Verbindung der allgemeinen Formel I oder II mit den für die Indices a, b, x und y angegebenen Bereichen. Besonders bevorzugt werden oberflächenaktive Mittel mit einem HLB-Wert zwischen 26 und 30 verwendet. Das oberflächenaktive Mittel ist in dem Reagenz in einer Konzentration von 0,1 bis 2 % enthalten.

Mit dem erfindungsgemäßen Verfahren und Reagenz gelingt es die Wiederfindung bei Immunbestimmungen in heterogener Phase zu verbessern. Durch den Zusatz des erfindungsgemäß verwendeten Mittels wird ferner die Immunreaktion beschleunigt. Darüberhinaus werden unerwünschte Nebenreaktionen unterdrückt wie insbesondere die unspezifische Anlagerung von Konjugat an die feste Phase, ohne daß es zu

einer verstärkten Ablösung der gebundenen Reaktionspartner kommt.

**Beispiel 1**

Es wurden Kunststoffröhrchen mit Antikörper gegen TSH, die mittels der Chloramin-T-Methode mit 125 J markiert waren, in Phosphatpuffer mit 40 mmol/l bei einem pH 7,4 beladen. Die Konzentration war 2 μg/ml. Nachbeladen wurde mit einer Lösung aus 0,9 % Natriumchlorid und 0,3 % Rinderserumalbumin, Typ II. Das Beladevolumen lag bei 1,5 ml. Die Inkubationszeit für die Beladung betrug 22 Stunden. Danach wurde mit 1,8 ml einer Nachbeladelösung 1/2 Stunde lang nachbeladen. Die Röhrchen wurden 24 Stunden getrocknet mit der Röhrchenöffnung nach oben. Die Röhrchen wurden dann mit Detergenz behandelt. Bei diesen Röhrchen wurde dann die Desorption des Antigens bzw. des Antigen-Antikörper- Komplexes gemessen. Die Ergebnisse sind der folgenden Tabelle I zu entnehmen.

T a b e l l e 1

| Detergenz | Konzen-tration | Desorption Wand-AK | Antigen |
|---|---|---|---|
| Pufferlösung | - | 3,2 % | 1,2 % |
| Tween® 20 (V) | 1 % | 58 % | 74 % |
| " | 0,1 % | 50 % | 65 % |
| " | 0,01 % | 30 % | 45 % |
| Triton® X 100 (V) | 1 % | 61 % | 85 % |
| " | 0,1 % | 62 % | 89 % |
| " | 0,01 % | 53 % | 78 % |
| Pluronic® L 64 (V) | 1 % | 64 % | 85 % |
| " | 0,1 % | 21 % | 39 % |
| " | 0,01 % | 6 % | 10 % |
| Pluronic® F 68 (E) | 1 % | 9 % | 7 % |
| " | 0,1 % | 5 % | 6 % |
| " | 0,01 % | 5 % | 5 % |
| Tetronic® 707 (E) | 1 % | 5 % | - |
| " | 0,1 % | 4 % | - |
| " | 0,01 % | 4 % | - |

V = Vergleich, oberflächenaktives Mittel mit HLB $<$ 20

E = erfindungsgemäßes oberflächenaktives Mittel mit HLB $>$ 20.

**Beispiel 2**

Es wurde die Wiederfindung im TSH-Test bei Verwendung verschiedener Detergenzien überprüft. Dazu wurde mit EDTA, mit Heparin bzw. mit Citrat stabilisiertes Plasma verwendet. Gleichzeitig wurde der TSH-Test mit Serum durchgeführt. Bei diesem Test war die Wiederfindung 100 %. Die für verschiedene Detergenzien erhaltenen Werte sind der folgenden Tabelle zu entnehmen:

Tabelle 2

| Detergenz | Detergenzkonzentration Inkubations-puffer | Konjugat-puffer | Wiederfindung EDTA Plasma | Heparin Plasma | Citrat Plasma |
|---|---|---|---|---|---|
| ohne | - | - | 40-70 | 40-70 | - |
| Triton® X 405 | 0,1 % | - | 66 % | 81 % | - |
| Tween® 20 | 0,005% | - | 88 % | 89 % | 89 % |
| Pluronic® F 68 | 1 % | - | 98 % | 97 % | 93 % |
| " | 1 % | 1 % | 105 % | 98 % | 99 % |
| Pluronic® F 87 | 1 % | - | 99 % | 105 % | 100 % |
| " | 1 % | 1 % | 106 % | 100 % | 112 % |
| Pluronic® F 88 | 1 % | - | 106 % | 93 % | - |
| " | 1 % | 1 % | 103 % | 92 % | - |
| | 1 % | - | 105 % | 104 % | 105 % |
| Pluronic® F 98 | 1 % | 1 % | 99 % | 98 % | 102 % |
| " | - | 2 % | 98 % | 98 % | 99 % |
| Pluronic® F 127 | 1 % | - | 103 % | 98 % | - |
| " | 1 % | 1 % | 101 % | 95 % | - |
| Pluronic® F 38 | 1 % | - | 102 % | 86 % | 94 % |
| Pluronic® F 108 | 1 % | 1 % | 105 % | 92 % | 101 % |
| Tetronic® 707 | 1 % | - | 100 % | 97 % | 105 % |
| " | 1 % | 1 % | 98 % | 94 % | 102 % |
| " | - | 2 % | 96 % | 83 % | 96 % |
| Tetronic® 908 | 1 % | - | 98 % | 94 % | 100 % |
| " | 1 % | 1 % | 97 % | 94 % | 99 % |
| Tetronic® 909 | 1 % | - | 90 % | 84 % | 95 % |
| " | 1 % | 1 % | 98 % | 96 % | 101 % |
| Tetronic® 1107 | 1 % | - | 110 % | 103 % | - |
| " | 1 % | 1 % | 105 % | 99 % | - |
| Tetronic® 1307 | 1 % | - | 105 % | 96 % | - |
| " | 1 % | 1 % | 107 % | 103 % | - |

Serumwert = 100 %

Analoge Versuche wurden auch im CEA-Test durchgeführt. Die Ergebnisse entsprachen denen im TSH-Test. Es konnte festgestellt werden, daß bei steigenden Konzentrationen an oberflächenaktivem Mittel nicht nur die Wiederfindung im Plasma steigt, sondern auch die Extinktion des Nullstandards erniedrigt wird. Das bedeutet, daß die unspezifische Konjugatbindung durch den Zusatz des oberflächenaktiven Mittels verringert wird.

**Beispiel 3**

Um den Einfluß von verschiedenen Detergenzien auf die unspezifische Bindung von Konjugat zu zeigen, wurden verschiedene oberflächenaktive Mittel getestet. Die Ergebnisse sind der folgenden Tabelle 3 zu entnehmen.

Tabelle 3

| Detergenz | Konzentration | Ext. 0-Standard |
|---|---|---|
| ohne | - | 68 mE |
| Tetronic® 707 | 1 % | 43 mE |
| " | 2 % | 26 mE |
| Tetronic® 908 | 1 % | 10 mE |
| Pluronic® F 68 | 2 % | 22 mE |
| Pluronic® F 88 | 2 % | 18 mE |

**Beispiel 4**

Es wurde geprüft, inwieweit das erfindungsgemäß verwendete oberflächenaktive Mittel den TSH-Test beschleunigen kann, ohne daß die Extinktion des Nullstandards sich erhöht. Dazu wurden verschiedene oberflächenaktive Mittel getestet. Die Ergebnisse sind der beigefügten Abbildung zu entnehmen.

**Beispiel 5**

Es wurden mehrere TSH-Tests durchgeführt. Dabei wurde die Beschleunigung, bezogen auf einen Standard, bestimmt, die mit den erfindungsgemäß verwendeten Mitteln erreicht werden kann. Die in der folgenden Tabelle 4 aufgeführten Werte wurden dabei mit den verschiedenen oberflächenaktiven Mitteln erhalten.

Tabelle 4

| oberflächenaktives Mittel | Konz. % | Standard ΔE | % |
|---|---|---|---|
| Pluronic® F 68 | 1 % | 0,847 | 100,5 |
| " | 2 % | 0,926 | 110 |
| " | 5 % | 1,238 | 147 |
| Pluronic® F 88 | 1 % | 0,775 | 114 |
| " | 2 % | 0,762 | 112 |
| " | 5 % | 1,002 | 147 |
| Pluronic® F 98 | 1 % | 0,831 | 118 |
| " | 2 % | 0,789 | 112 |
| " | 5 % | 1,213 | 172 |
| Pluronic® F 38 | 1 % | 0,697 | 102 |
| " | 2 % | 0,695 | 102 |
| " | 5 % | 0,627 | 92 |

## Ansprüche

1. Verfahren zur Bestimmung eines Partners einer Immunreaktion nach dem Immunoassay-Prinzip bei einer Temperatur zwischen 15 und 40° C in Gegenwart eines oberflächenaktiven Mittels, wobei einer der Reaktionspartner in fester Phase vorliegt, **dadurch gekennzeichnet,** daß man als oberflächenaktives Mittel nichtionische Block-Copolymere auf Basis von Alkylenoxiden mit 2 bis 4 C-Atomen, die gegebenenfalls ein Alkylendiamin mit 2 bis 4 C-Atomen als Zentralmolekül enthalten, mit einem HLB-Wert von mehr als 20 in einer Menge von 0,1 bis 2 Gew.-%, bezogen auf das Gewicht des Reaktionsansatzes zusetzt.

2. Verfahren nach Anspruch 1, **dadurch gekennzeichnet,** daß die Bestimmung in einer Plasmaprobe durchgeführt wird.

3. Verfahren nach einem der Ansprüche 1 oder 2, **dadurch gekennzeichnet,** daß man oberflächenaktive Mittel mit einem HLB-Wert zwischen 24 und 30 verwendet.

4. Verfahren nach einem der Ansprüche 1 bis 3, **dadurch gekennzeichnet,** daß man als oberflächenaktive Mittel Verbindungen der Formel I

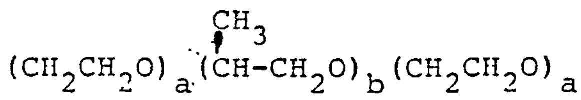

worin a einen Wert zwischen 30 bis 150 und b einen Wert zwischen 15 und 60 annehmen kann, einsetzt.

5. Verfahren nach Anspruch 4 , **dadurch gekennzeichnet,** daß man Blockcopolymere einsetzt, bei denen a einen Wert zwischen 60 und 100 und b einen Wert zwischen 25 und 40 hat.

6. Verfahren nach einem der Ansprüche 3 bis 5, **dadurch gekennzeichnet,** daß man Blockcopolymere einsetzt, die einen Anteil an hydrophilen Polyoxyethylengruppen von 60 bis 90 % aufweisen.

7. Verfahren nach einem der Ansprüche 1 bis 3, **dadurch gekennzeichnet,** daß man als oberflächenaktive Mittel Verbindungen mit der folgenden Formel II einsetzt:

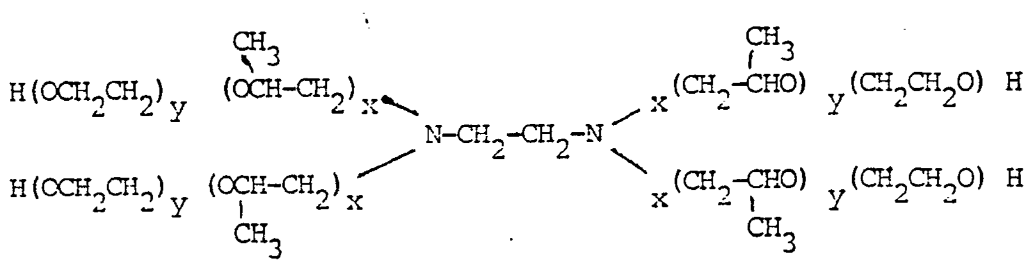

worin x einen Wert zwischen 10 und 50 und y einen Wert zwischen 30 und 150 annehmen kann.

8. Verfahren nach Anspruch 7, **dadurch gekennzeichnet,** daß man Blockcopolymere einsetzt, bei denen x einen Wert zwischen 15 und 30 und y einen Wert zwischen 40 und 120 hat.

9. Verfahren nach einem der Ansprüche 7 oder 8, **dadurch gekennzeichnet,** daß der Anteil an Polyoxyethylengruppen 50 bis 90 % beträgt.

10. Verfahren nach einem der vorhergehenden Ansprüche, **dadurch gekennzeichnet,** daß das oberflächenaktive Mittel zusammen mit der Inkubationslösung eingebracht wird.

11. Reagenz zur Durchführung eines Verfahrens nach einem der Ansprüche 1 bis 10, enthaltend mobilisierte und immobilisierte Partner einer Immunreaktion sowie ein oberflächenaktives Mittel, **dadurch gekennzeichnet,** daß es als oberflächenaktives Mittel nichtionische Block-Copolymere auf Basis von Alkylenoxiden mit 2 bis 4 C-Atomen, die gegebenenfalls ein Alkylendiamin mit 2 bis 4 C-Atomen als Zentralmolekül enthalten, mit einem HLB-Wert von mehr als 20 in einer Menge von 0,1 bis 2 Gew.-% enthält.

12. Reagenz nach Anspruch 11, **dadurch gekennzeichnet,** daß das oberflächenaktive Mittel einen HLB-Wert zwischen 24 und 30 hat.

13. Reagenz nach einem der Ansprüche 11 oder 12, **dadurch gekennzeichnet,** daß das oberflächenaktive Mittel eine Verbindung mit der Formel I

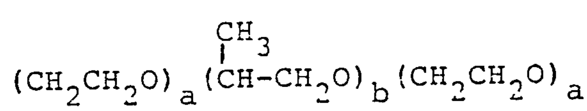

ist, worin a einen Wert zwischen 30 und 150, bevorzugt zwischen 60 und 100 und b einen Wert zwischen 15 und 60, bevorzugt zwischen 25 und 40 hat.

14. Reagenz nach Anspruch 13, **dadurch gekennzeichnet,** daß die Verbindung einen Anteil an Polyoxyethylengruppen von 60 bis 90 % hat.

15. Reagenz nach einem der Ansprüche 11 oder 12, **dadurch gekennzeichnet,** daß das oberflächenaktive Mittel eine Verbindung mit der allgemeinen Formel II

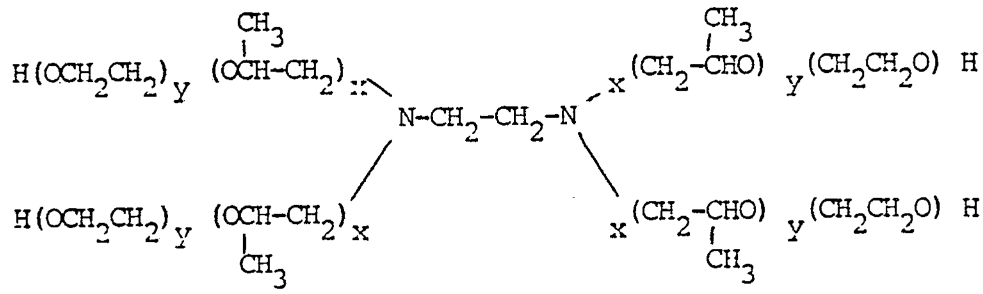

ist, worin x einen Wert zwischen 10 und 50, bevorzugt zwischen 15 und 30 und y einen Wert zwischen 30 und 150, bevorzugt zwischen 40 und 120 hat.

16. Reagenz nach Anspruch 15, **dadurch gekennzeichnet,** daß die Verbindung einen Anteil an Polyoxyethylengruppen von 50 bis 90 % hat.

17. Reagenz nach einem der vorhergehenden Ansprüche, **dadurch gekennzeichnet,** daß es noch ein System zum Nachweis einer als Markierung verwendeten Enzymaktivität enthält.

**Claims**

1. Process for the determination of a partner of an immune reaction according to the immunoassay principle at a temperature between 15 and 40° C. in the presence of a surface-active agent, whereby one of the reaction partners is present in solid phase, characterised in that, as surface-active agent, one adds non-ionic block polymers based on alkylene oxides with 2 to 4 C-atoms, which possibly contain an alkylenediamine with 2 to 4 C-atoms as central molecule, with an HLB value of more than 20 in an amount of 0.1 to 2 wt.%, referred to the weight of the reaction batch.

2. Process according to claim 1, characterised in that the determination is carried out in a plasma sample.

3. Process according to one of claims 1 or 2, characterised in that one uses surface-active agents with an HLB value between 24 and 30.

4. Process according to one of claims 1 to 3, characterised in that, as surface-active agents, one uses compounds of the formula I

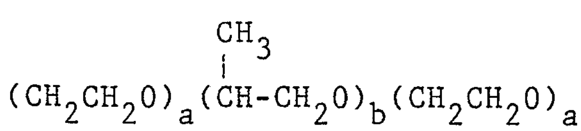

wherein a can have a value between 30 and 150 and b a value between 15 and 60.

5. Process according to claim 4, characterised in that one uses block co-polymer in which a has a value between 60 and 100 and b a value between 25 and 40.

6. Process according to one of claims 3 to 5, characterised in that one uses block co-polymers which have a proportion of hydrophilic polyoxyethylene groups of 60 to 90%.

**7.** Process according to any of claims 1 to 3, characterised in that, as surface-active agents, one uses compounds with the following formula II

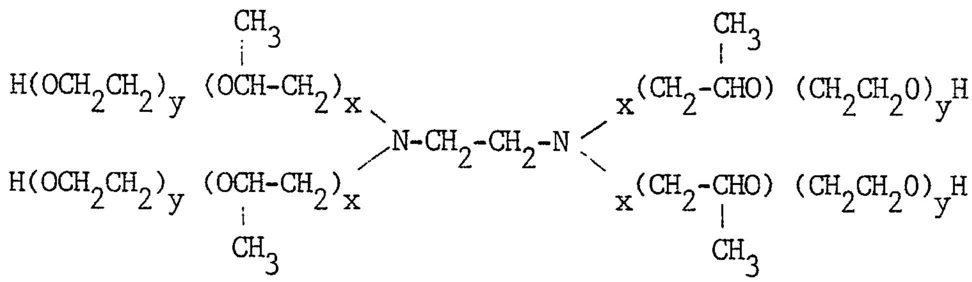

wherein $x$ can have a value between 10 and 50 and $y$ a value between 30 and 150.

**8.** Process according to claim 7, characterised in that one uses block co-polymers in which $x$ has a value between 15 and 30 and $y$ a value between 40 and 120.

**9.** Process according to one of claims 7 or 8, characterised in that the proportion of polyoxyethylene groups amounts to 50 to 90%.

**10.** Process according to one of the preceding claims, characterised in that the surface-active agent is introduced together with the incubation solution.

**11.** Reagent for the carrying out of a process according to one of claims 1 to 10, containing mobilised and immobilised partners of an immune reaction, as well as a surface-active agent, characterised in that, as surface-active agent, it contains non-ionic block co-polymers based on alkylene oxides with 2 to 4 C-atoms which optionally contain an alkylenediamine with 2 to 4 C-atoms as the central molecule, with an HLB value of more than 20, in an amount of 0.1 to 2 wt.%.

**12.** Reagent according to claim 11, characterised in that the surface-active agent has an HLB value between 24 and 30.

**13.** Reagent according to one of claims 11 or 12, characterised in that the surface-active agent is a compound of the formula I

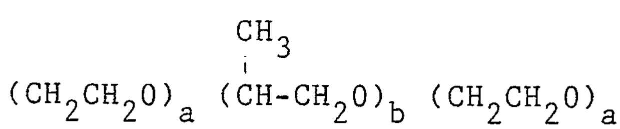

wherein $a$ has a value between 30 and 150, preferably between 60 and 100, and $b$ has a value between 15 and 60, preferably between 25 and 40.

**14.** Reagent according to claim 13, characterised in that the compound has a proportion of polyoxyethylene groups of 60 to 90%.

**15.** Reagent according to one of claims 11 or 12, characterised in that the surface-active agent is a compound with the general formula II

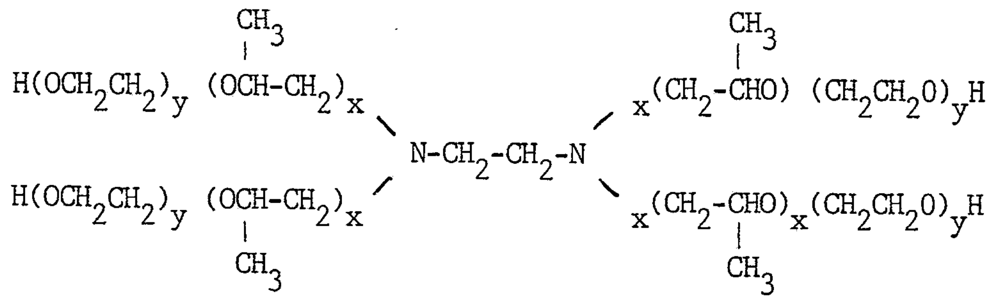

wherein x has a value between 10 and 50, preferably between 15 and 30, and y has a value between 30 and 150, preferably between 40 and 120.

16. Reagent according to claim 15, characterised in that the compound has a proportion of polyoxyethylene groups of 50 to 90%.

17. Reagent according to one of claims 11 to 16, characterised in that it also contains a system for the detection of an enzyme activity used as labelling.

**Revendications**

1. Procédé pour la détermination d'un coréactant ou partenaire d'une réaction immunologique selon le principe de l'immuno-essai à une température entre 15 et 40° C en présence d'un agent tensioactif, dans lequel l'un des partenaires de la réaction se présente sous forme solide, caractérisé en ce que l'on ajoute, comme agent tensioactif, des copolymères blocs non ioniques à base d'oxydes d'alkylène avec 2 à 4 atomes de C qui contiennent éventuellement une alkylène diamine avec 2 à 4 atomes de C comme molécule centrale, avec une valeur d'équilibre hydrophile-lipophile (HLB) supérieure à 20 en une quantité de 0,1 à 2 % en poids, par rapport au poids du mélange réactionnel.

2. Procédé selon la revendication 1, caractérisé en ce que la détermination est effectuée dans un échantillon de plasma.

3. Procédé selon l'une des revendications 1 ou 2, caractérisé en ce que l'on utilise des agents tensioactifs présentant une valeur d'équilibre hydrophile-lipophile (HLB) comprise entre 24 et 30.

4. Procédé selon l'une des revendications 1 à 3, caractérisé en ce que l'on utilise, comme agents tensioactifs, des composés de formule I

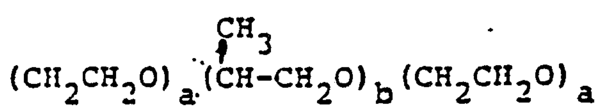

dans laquelle a peut prendre une valeur comprise entre 30 et 150 et b une valeur comprise entre 15 et 60.

5. Procédé selon la revendication 4, caractérisé en ce que l'on met en oeuvre des copolymères blocs dans lesquels a a une valeur comprise entre 60 et 100 et b une valeur comprise entre 25 et 40.

6. Procédé selon l'une des revendications 3 à 5, caractérisé en ce que l'on met en oeuvre des copolymères blocs qui présentent une proportion de groupes polyoxyéthylène hydrophiles de 60 à 90 %.

7. Procédé selon l'une des revendications 1 à 3, caractérisé en ce que l'on met en oeuvre, comme agents tensioactifs, des composés présentant la formule II suivante :

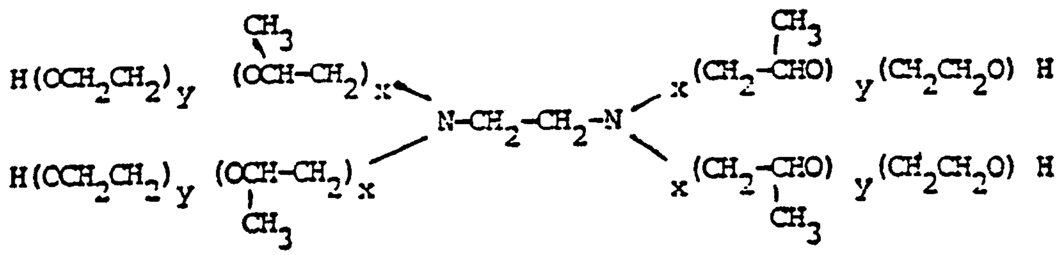

dans laquelle x peut prendre une valeur comprise entre 10 et 50 et y une valeur comprise entre 30 et 150.

8. Procédé selon la revendication 7, caractérisé en ce que l'on met en oeuvre des copolymères blocs dans lesquels x a une valeur comprise entre 15 et 30 et y une valeur comprise entre 40 et 120.

9. Procédé selon l'une des revendications 7 ou 8, caractérisé en ce que la proportion des groupes polyoxyéthylène est de 50 à 90 %.

10. Procédé selon l'une des revendications précédentes, caractérisé en ce que l'agent tensioactif est introduit ensemble avec la solution d'incubation.

11. Réactif pour la mise en oeuvre d'un procédé selon l'une des revendications 1 à 10, contenant des partenaires mobilisés et immobilisés d'une réaction immunologique ainsi qu'un agent tensioactif, caractérisé en ce qu'il contient, en tant qu'agent tensioactif, des copolymères blocs non ioniques à base d'oxydes d'alkylène avec 2 à 4 atomes de C qui contiennent éventuellement une alkylènediamine avec 2 à 4 atomes de C comme molécule centrale, avec une valeur d'équilibre hydrophile-lipophile (HLB) supérieure à 20 en une quantité de 0,1 à 2 % en poids.

12. Réactif selon la revendication 11, caractérisé en ce que l'agent tensioactif a une valeur d'équilibre hydrophile-lipophile (HLB) comprise entre 24 et 30.

13. Réactif selon l'une des revendications 11 ou 12, caractérisé en ce que l'agent tensioactif est un composé de formule I

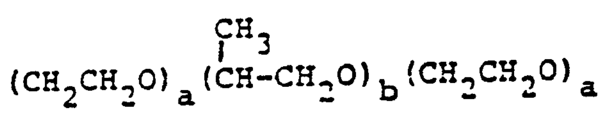

dans laquelle a a une valeur comprise entre 30 et 150, de préférence entre 60 et 100 et b une valeur comprise entre 15 et 60, de préférence entre 25 et 40.

14. Réactif selon la revendication 13, caractérisé en ce que le composé comporte une proportion de groupes polyoxyéthylène de 60 à 90 %.

15. Réactif selon l'une des revendications 11 ou 12, caractérisé en ce que l'agent tensioactif est un composé de formule générale II

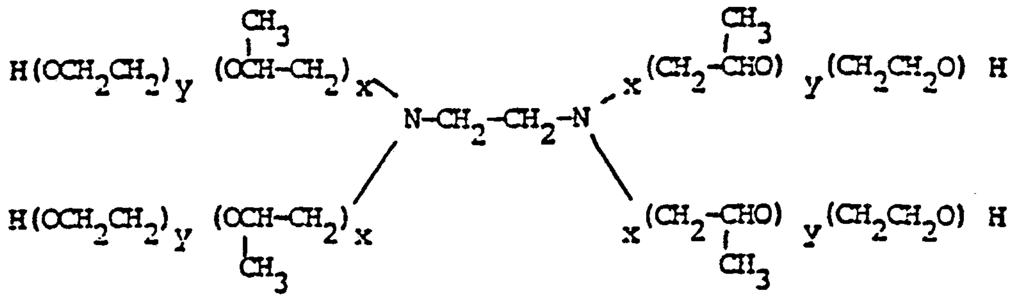

dans laquelle x a une valeur comprise entre 10 et 50, de préférence entre 15 et 30 et y une valeur comprise entre 30 et 150, de préférence entre 40 et 120.

16. Réactif selon la revendication 15, caractérisé en ce que le composé présente une proportion de groupes polyoxyéthylène de 50 à 90 %.

17. Réactif selon l'une des revendications précédentes, caractérisé en ce qu'il contient en outre un système pour la mise en évidence d'une activité enzymatique utilisée comme marquage.

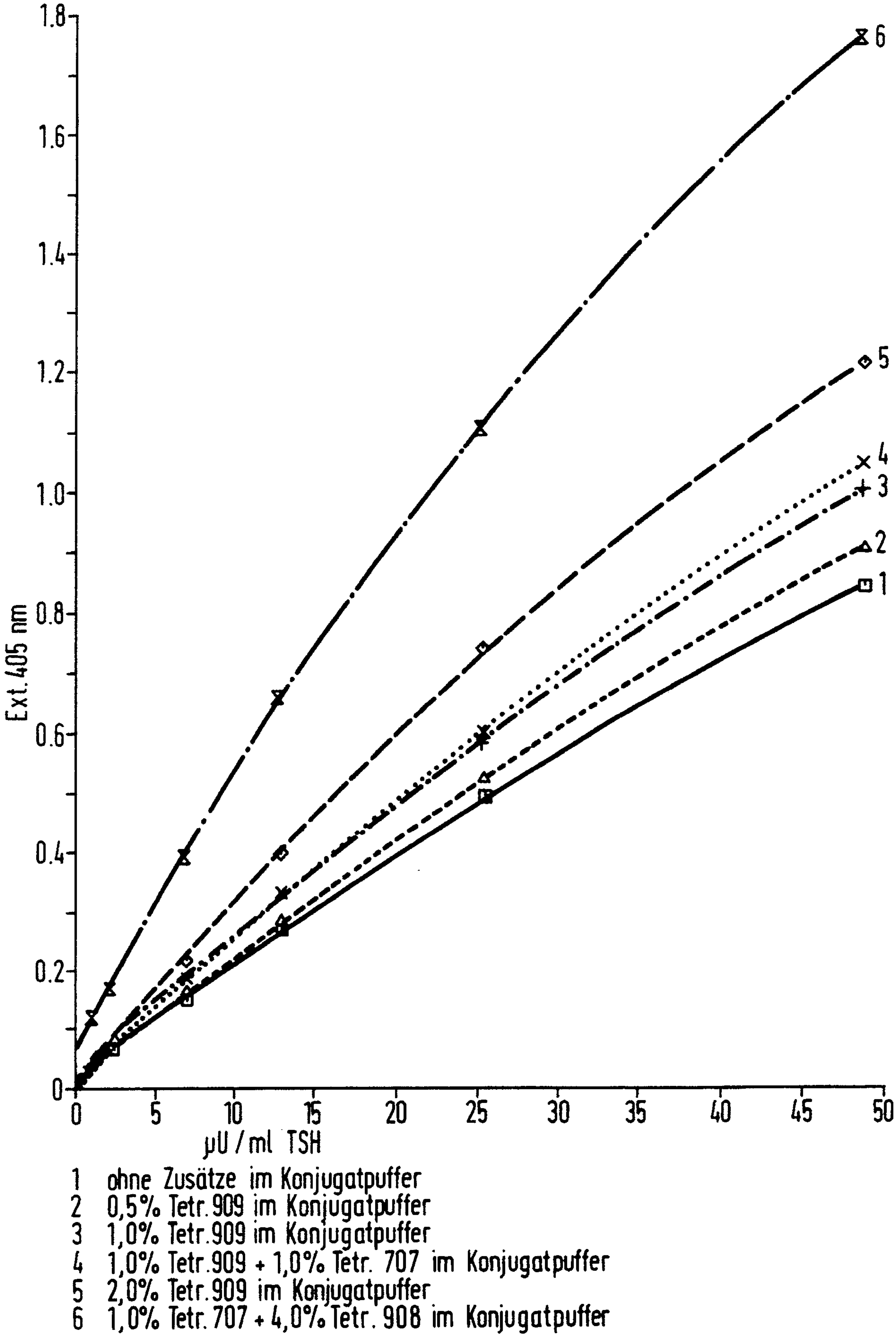


1 ohne Zusätze im Konjugatpuffer
2 0,5% Tetr.909 im Konjugatpuffer
3 1,0% Tetr.909 im Konjugatpuffer
4 1,0% Tetr.909 + 1,0% Tetr. 707 im Konjugatpuffer
5 2,0% Tetr.909 im Konjugatpuffer
6 1,0% Tetr. 707 + 4,0% Tetr. 908 im Konjugatpuffer